# EUROPÄISCHE PATENTSCHRIFT

(11) **EP 2 484 454 B1**
(45) Veröffentlichungstag und Bekanntmachung des Hinweises auf die Patenterteilung: **14.05.2014**
(21) Anmeldenummer: 11153081.2
(22) Anmeldetag: 02.02.2011
(51) Int. Cl.: B05B 11/02, B05B 1/34, A61J 1/20, A61J 1/06, A61M 15/08, A61M 5/19, A61M 5/24, A61M 11/00

(54) **Austragvorrichtung für fließfähigen Stoff**
Discharge device for flowable material
Dispositif de sortie pour matière s'écoulant

(43) Veröffentlichungstag der Anmeldung: 08.08.2012
(73) Patentinhaber: Dispensys AG, 9100 Herisau (CH)
(72) Erfinder: Sògaro, Alberto C., 61476 Kronberg (DE)
(74) Vertreter: Manitz, Finsterwald & Partner GbR

(56) Entgegenhaltungen:
- EP-A1- 1 504 783
- EP-A1- 2 011 467
- DE-U1- 20 019 091
- GB-A- 2 403 153

## Beschreibung

Die Erfindung betrifft eine Austragvorrichtung für fließfähigen Stoff, insbesondere für ein Medikament und/oder einen Impfstoff, mit den Merkmalen des Oberbegriffs des Patentanspruchs 1.

Eine derartige Austragvorrichtung ist aus der DE 200 19 091 U1-bekannt und dient insbesondere zum Applizieren pharmazeutischer oder kosmetischer Substanzen am menschlichen Körper. Die bekannte Appliziervorrichtung ist nach Art einer Minispritze ausgebildet und weist einen Trägerkörper auf, der stirnseitig mit einer Austragöffnung zum Austragen des fließfähigen Stoffes versehen ist. An der der Austragöffnung abgewandten Seite weist der Trägerkörper einen zylindrischen Zapfen auf, der einen Axialkanal und einen von dem Axialkanal abzweigenden Querkanal aufweist. Auf dem zylindrischen Zapfen, das heißt an dessen Außenseite ist ein Speicherelement über zwei axial voneinander beabstandete Dichtlippen geführt. Das Speicherelement ist topfartig bzw. nach Art eines einseitig geschlossenen Röhrchens ausgebildet und kann auf dem zylindrischen Zapfen manuell so verschoben werden, dass der Zapfen als Verdrängungskolben wirkt, der die in dem Speicherelement vorgehaltene fließfähige Substanz verdrängt und über den Querkanal und den Axialkanal des Trägerkörpers zu der Austragöffnung fördert. Der fließfähige Stoff kann über die Austragöffnung ausgesprüht werden.

Mittels der aus der Druckschrift DE 200 19 091 U1 bekannten Austragvorrichtung ist der fließfähige Stoff gegebenenfalls nicht hinreichend zerstäubt austragbar.

Der Erfindung liegt die Aufgabe zugrunde, eine gemäß der einleitend genannten Gattung ausgebildete Appliziervorrichtung zu schaffen, die sich gegenüber dem Stand der Technik durch verbesserte Zerstäubungseigenschaften auszeichnet.

Diese Aufgabe ist erfindungsgemäß durch die Austragvorrichtung mit den Merkmalen des Patentanspruchs 1 gelöst.

Gemäß der Erfindung umfasst der Trägerkörper mithin im Bereich der Austragöffnung eine Zerstäubungseinrichtung, mittels der der fließfähige Stoff durch Verschieben des Speicherelements in zerstäubter Form ausgetragen wird.

Die Austragvorrichtung nach der Erfindung kann insbesondere zum Applizieren eines Medikaments oder eines Impfstoffes in ein Nasenloch eines Menschen oder auch zum Applizieren eines Medikaments oder eines Impfstoffes auf die Rachenschleimhaut eines Menschen genutzt werden. Grundsätzlich eignet sich die Austragvorrichtung nach der Erfindung für Ein- oder Mehrkomponentensysteme, die bei der Applikation einer Zerstäubung bzw. Zerwellung unterzogen werden sollen.

Bei der Austragvorrichtung nach der Erfindung umfasst die Zerstäubungseinrichtung eine Düse, die stromab eines Zerstäubungsraums angeordnet ist, in dem ein Ablenkelement angeordnet ist, so dass fließfähiger Stoff in zerstäubter Form über die Düse ausgetragen wird. Das Ablenkelement wirkt hierbei derart als Leiteinrichtung, dass der fließfähige Stoff bezogen auf die Achse der Düse radial nach außen versetzt zu der Düse gefördert wird. Der fließfähige Stoff wird also in dem Zerstäubungsraum damit außermittig zur Düse gefördert, was die Zerstäubungseigenschaften der Austragvorrichtung verbessert.

Bei einer speziellen Ausführungsform der Austragvorrichtung nach der Erfindung ist das Ablenkelement aus einem plättchenartigen oder kugelartigen Element gebildet, an dessen Umfang der fließfähige Stoff in Richtung der Düse gefördert wird. Der Umfang des derartig ausgebildeten Ablenkelements definiert zusammen mit der Wand des Zerstäubungsraums den Strömungsweg des fließfähigen Stoffs.

Zur weiteren Optimierung des Zerstäubungsverhaltens ist bei der Austragvorrichtung nach der Erfindung stromauf des Zerstäubungsraums ein Austragkanal angeordnet, in den ein Reduzierstab eingreift. Der Reduzierstab bildet zusammen mit der Wand des Austragkanals einen Ringkanal, durch den der fließfähige Stoff zu dem Zerstäubungsraum transportiert wird. Der Reduzierstab dient auch dazu, nach Anwendung der Austragvorrichtung möglichst wenige Rückstände in der Austragvorrichtung zu belassen. Der Reduzierstab begrenzt also das Volumen des Austragkanals.

Um eine leichte Montierbarkeit der Austragvorrichtung nach der Erfindung zu gewährleisten, kann der Reduzierstab einstückig mit dem Ablenkelement ausgebildet sein.

Bei einer herstellungstechnisch kostengünstig umzusetzenden Ausführungsform ist der zylindrische Zapfen von einem Einsatz gebildet, der an dem Trägerkörper insbesondere über einen Ringbund verrastet ist.

Der Einsatz, der den zylindrischen Zapfen bildet, ist vorzugsweise von einer Seite in den Trägerkörper eingesetzt. Von der dem Einsatz abgewandten Seite ist dann zweckmäßigerweise der mit dem Ablenkelement versehene Reduzierstab durch die Austragöffnung und den Austragkanal eingeschoben, so dass das Ablenkelement innerhalb des Zerstäubungsraums angeordnet ist. Zur Fixierung des Reduzierstabs kann ein topfartiger Düseneinsatz dienen, der den Zerstäubungsraum radial begrenzt und an dem stirnseitig die Düse ausgebildet ist.

Insbesondere bei einer Austragvorrichtung, die für ein Einkomponentensystem ausgebildet ist und einen Aufnahmeraum für fließfähigen Stoff und einen zylindrischen Zapfen aufweist, und bei der der Axialkanal des Zapfens mit dem sich an den Zerstäubungsraum anschließenden Austragkanal fluchtet, kann der Reduzierstab in den Axialkanal des Zapfens eingreifen und diesen vorzugsweise weitgehend durchgreifen.

Des Weiteren kann der Trägerkörper zur Fixierung des Einsatzes einen axialen Durchgang aufweisen, der gestuft ausgebildet ist und in den der Einsatz eingreift. Der Austragkanal kann Teil des Durchgangs sein oder in diesen übergehen.

Bei einer Austragvorrichtung für ein Mehrkomponentensystem oder für fließfähigen Stoff, der in mehreren Aufnahmeräumen des Speicherelements vorgehalten ist, können an dem Einsatz mindestens zwei der Zapfen ausgebildet sein, an denen jeweils ein Speicherelement verschiebbar geführt ist oder an denen ein einziges Speicherelement mit einer entsprechenden Anzahl an Aufnahmeräumen verschiebbar geführt ist. In jedem Falle greift jeweils ein Zapfen in einen Aufnahmeraum ein. Es können auch mehrere Einsätze mit jeweils einem Zapfen vorgesehen sein.

Zwischen dem Einsatz mit mehreren Zapfen und dem Trägerkörper kann ein Leitstück angeordnet sein, das zumindest teilweise den zu dem Zerstäubungsraum führenden Austragkanal bildet. In dem Leitstück, das ebenfalls in den Trägerkörper eingesetzt ist, kann bei einem Mehrkomponentensystem die Mischung der Komponenten erfolgen. Von dem Leitstück wird der auszutragende Stoff gegebenenfalls in gemischter Form zu dem Austragkanal geführt.

Um das Applizieren des fließfähigen Stoffes zu erleichtern, ist an dem Trägerkörper vorzugsweise mindestens eine Fingeranlagefläche ausgebildet oder weist das Speicherelement eine Drückerfläche auf, die an einer Drückerplatte ausgebildet sein kann. Die Austragvorrichtung nach der Erfindung kann damit nach Art einer handelsüblichen Einwegspritze betätigt werden, so dass bei deren Benutzung zwei Finger eines Nutzers an den Fingeranlageflächen des Trägerkörpers anliegen und der Daumen des Nutzers auf die Drückerfläche des Speicherelements drückt, so dass die Austragvorrichtung teleskopiert, das heißt zusammengefahren wird und der bzw. die zylindrischen Zapfen den fließfähigen Stoff aus dem bzw. den Aufnahmeräumen nach Art eines Verdrängerkolbens austragen.

Weitere Vorteile und vorteilhafte Ausgestaltungen des Gegenstandes der Erfindung sind der Beschreibung, der Zeichnung und den Patentansprüchen entnehmbar.

Drei Ausführungsbeispiele einer Austragvorrichtung nach der Erfindung sind in der Zeichnung schematisch vereinfacht dargestellt und werden in der nachfolgenden

Beschreibung näher erläutert. Es zeigt:
- Fig. 1: eine perspektivische Ansicht einer Austragvorrichtung mit einem Aufnahmeraum für fließfähigen Stoff;
- Fig. 2: einen Längsschnitt durch die Austragvorrichtung nach Fig. 1;
- Fig. 3: einen Längsschnitt durch einen Trägerkörper der Austragvorrichtung in Alleinstellung;
- Fig. 4: einen Längsschnitt durch ein Speicherelement der Austragvorrichtung in Alleinstellung;
- Fig. 5: eine Stimansicht des Speicherelements;
- Fig. 6: einen Längsschnitt durch einen Einsatz des Trägerkörpers;
- Fig. 7: eine Seitenansicht eines Reduzierstabs der Austragvorrichtung;
- Fig. 8: eine Stimansicht des Reduzierstabs gemäß Pfeil VIII in Fig. 7;
- Fig. 9: eine perspektivische Ansicht einer zweiten Ausführungsform einer Austragvorrichtung mit zwei Aufnahmeräumen für fließfähigen Stoff;
- Fig. 10: einen Längsschnitt durch die Austragvorrichtung nach Fig. 9;
- Fig. 11: einen Längsschnitt durch einen Trägerkörper der Austragvorrichtung nach Fig. 9;
- Fig. 12: eine perspektivische Ansicht eines Leitstücks der Austragvorrichtung nach Fig. 9;
- Fig. 13: eine Stimansicht des Leitstücks;
- Fig. 14: einen Schnitt durch das Leitstück nach Fig. 12 entlang der Linie A-A in Fig. 13;
- Fig. 15: einen Schnitt entlang der Linie B-B in Fig. 13;
- Fig. 16: eine perspektivische Ansicht eines Einsatzes der Austragvorrichtung nach Fig. 9;
- Fig. 17: eine Stirnansicht des Einsatzes nach Fig. 16;
- Fig. 18: einen Schnitt durch den Einsatz nach Fig. 16 entlang der Linie A-A in Fig. 17;
- Fig. 19: einen Schnitt durch den Einsatz nach Fig. 16 entlang der Linie B-B in Fig. 17;
- Fig. 20: einen ersten Schnitt durch ein Speicherelement der Austragvorrichtung nach Fig. 9 mit zwei Aufnahmeräumen;
- Fig. 21: einen zweiten Schnitt durch das Speicherelement nach Fig. 20;
- Fig. 22: eine Seitenansicht eines Reduzierstabs der Austragvorrichtung nach Fig. 9;
- Fig. 23: eine Stimansicht des Reduzierstabs gemäß Pfeil X in Fig. 22;
- Fig. 24: eine perspektivische Ansicht einer dritten Ausführungsform einer Austragvorrichtung nach der Erfindung; und
- Fig. 25: einen Längsschnitt durch die Austragvorrichtung nach Fig. 24.

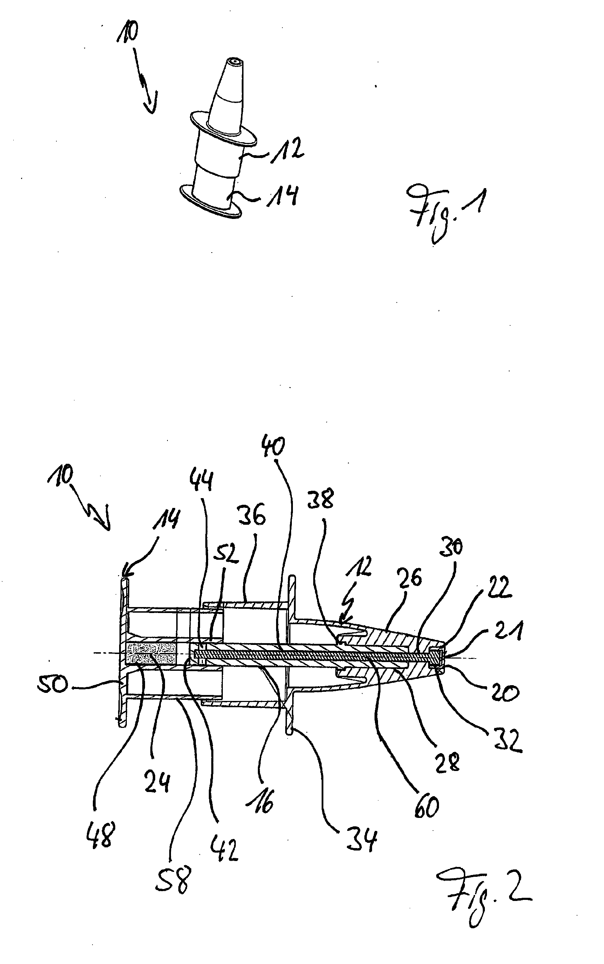

In den Figuren 1 bis 8 ist eine Austragvorrichtung 10 dargestellt, die spritzenartig ausgebildet ist und zur Applikation eines flüssigen Medikaments bzw. eines Impfstoffes in die menschliche Nase dient.

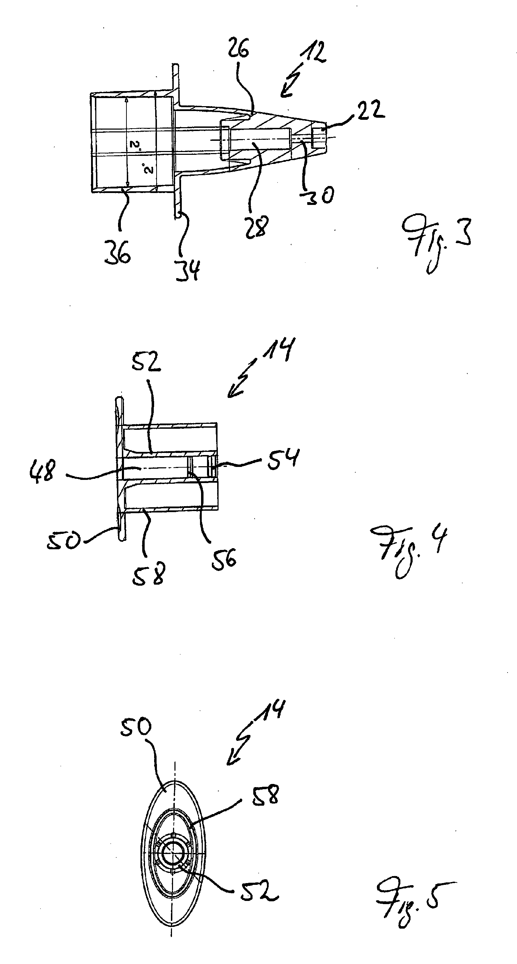

Die Austragvorrichtung 10 umfasst einen Trägerkörper bzw. Spritzenkörper 12, der in Fig. 3 in Alleinstellung dargestellt ist, und ein als Drücker ausgebildetes Speicherelement 14, welches in den Figuren 4 und 5 in Alleinstellung dargestellt ist. Als weitere Bauteile umfasst die Austragvorrichtung 10 einen stabförmigen bzw. im Wesentlichen zylinderförmigen Einsatz 16, einen Reduzierstab 60 sowie einen Düseneinsatz 20, der im Bereich einer Austragöffnung 22 des Trägerkörpers 12 eine Düse 21 zum Austragen des in dem Speicherelement 14 vorgehaltenen Medikaments 24 umfasst.

Der Trägerkörper 12 weist einen Sockel 26 auf, der einen sich in Richtung der Austragöffnung 22 verjüngenden, weitgehend elliptischen Querschnitt hat und in axialer Richtung von einem gestuft ausgebildeten Durchgang 28 durchsetzt ist. Der Durchgang 28 weist einen Austragkanal 30 auf, der in einen Zerstäubungsraum 32 mündet. In dem Zerstäubungsraum 32 ist im montierten Zustand der topfartig ausgebildete Düseneinsatz 20 fixiert, so dass dessen Umfangswand den Zerstäubungsraum 32 radial begrenzt.

An der dem Zerstäubungsraum 32 abgewandten Seite ist der Sockel 26 von einer Fingeranlageplatte 34 begrenzt, deren Ebene rechtwinkelig zur Achse des Trägerkörpers 12 ausgerichtet ist. An die Fingeranlageplatte 34 grenzt wiederum eine Führungswand 36 an, die sich in axialer Richtung des Trägerkörpers 12 erstreckt und ebenfalls eine im Wesentlichen elliptische Form mit konstantem Durchmesser aufweist.

In den Durchgang 28 des Sockels 26 ist von der dem Düseneinsatz 20 abgewandten Seite der zylindrische Einsatz 16 eingeführt und über einen Ringbund 38 verrastet, der mit der Stufung des Durchgangs 28 korrespondiert.

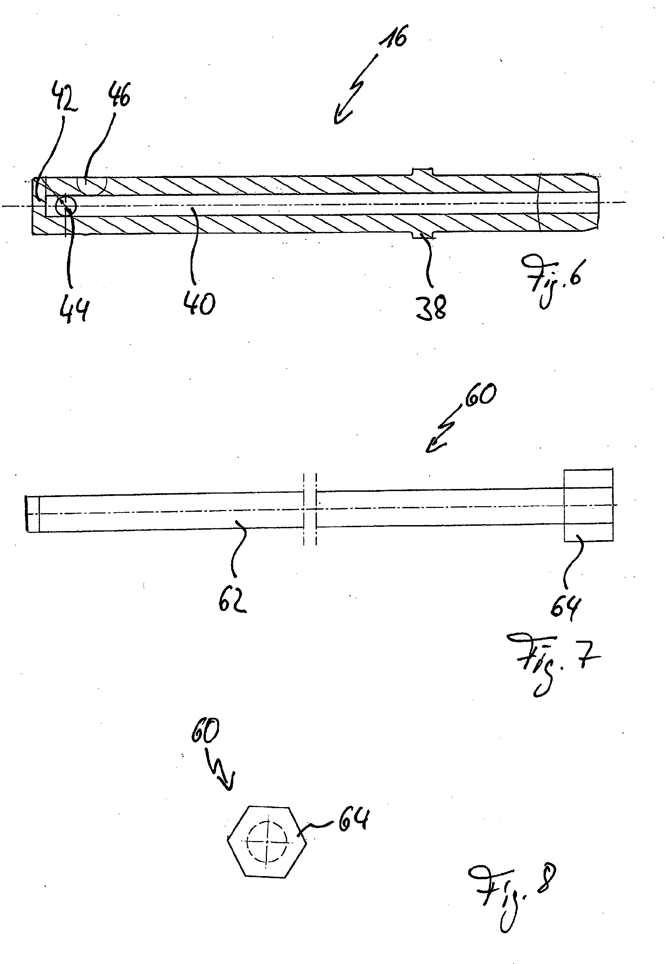

Der Einsatz 16, der nach Art eines Rohrs mit einseitig verschlossenem Ende ausgebildet ist, weist, wie insbesondere Fig. 6 zu entnehmen ist, einen Axialkanal 40 auf, der sich von der dem Zerstäubungsraum 32 zugewandten Stirnseite bis zu einer Stirnwand 42 erstreckt und von dem in dem an die Stirnwand 42 angrenzenden Endbereich ein Querkanal 44 abzweigt, der die Wand des Einsatzes 16 durchbricht. Des Weiteren ist am Umfang des Einsatzes 16 bezogen auf den Querkanal 44 in Richtung des Zerstäubungsraums 32 versetzt eine ringförmige Lippe 46 angeordnet, an der das als Drücker ausgebildete Speicherelement 14 geführt ist.

Wie bereits vorstehend ausgeführt, ist das Speicherelement 14 als Drücker ausgebildet, so dass durch dessen Betätigung das flüssige Medikament 24, das in einem Aufnahmeraum 48 des Speicherelements 14 vorgehalten ist, aus der Austragvorrichtung 10 ausgebracht werden kann.

Der Aufnahmeraum 48 ist stirnseitig einerseits von einer Drückerplatte 50 des Speicherelements 14 und im montierten Zustand andererseits von der Stirnwand 42 des Einsatzes 16 begrenzt. Radial ist der Aufnahmeraum 48 von einer Innenwand 52 begrenzt, die einen kreisförmigen Grundriss hat und an deren Innenseite zwei in axialer Richtung des Einsatzes 16 voneinander beabstandete Dichtlippen 54 und 56 angeordnet sind, deren Abstand etwas größer ist als der Durchmesser des Querkanals 44 des Einsatzes 16. Über die Dichtlippen 54 und 56 sowie die Lippe 46 des Einsatzes 16 ist das Speicherelement 14 verschiebbar an dem Einsatz 16 geführt. Hierbei können die Dichtlippen 54 und 56 des Speicherelements 14 einerseits und die Lippe 46 des Einsatzes 16 andererseits als Anschläge wirken, die dem Nutzer die jeweilige Betriebsstellung des Speicherelements 14 gegenüber dem Einsatz 16 vermitteln können. Jedoch können die Dichtlippen 54 und 56 beim Betätigen des Speicherelements 14 die Lippe 46 des Einsatzes 16 überfahren.

Das Speicherelement 14 weist des Weiteren eine Außenwand 58 auf, die einen elliptischen Grundriss hat und die Innenwand 52 umschließt und beim Betätigen des Speicherelements 14 an der Innenseite der Führungswand 36 des Trägerkörpers 12 geführt ist bzw. mit geringfügigem Abstand zu der Führungswand 36 verschoben wird.

Die Austragvorrichtung 10 weist des Weiteren einen Reduzierstab 60 auf, der in den Figuren 7 und 8 in Alleinstellung dargestellt ist und der einen zylindrischen Schaft 62 aufweist, der den Austragkanal 30 des Trägerkörpers 12 und den Axialkanal 40 des Einsatzes 16 vollständig durchgreift. Der Durchmesser des Schaftes 62 des Reduzierstabes 60 ist geringfügig kleiner als der Durchmesser des Axialkanals 40 des Einsatzes 16 und des Austragkanals 30 des Trägerkörpers 12.

An seinem dem Einsatz 16 abgewandten Ende weist der Reduzierstab 60 einen plättchenförmigen Kopf 64 auf, der nach Art eines hexagonalen Prismas ausgebildet ist und ein Ablenkelement für das aus der Austragvorrichtung auszutragende Medikament bildet. Der Kopf 64 ist innerhalb des Zerstäubungsraums 32 angeordnet, so dass er zusammen mit dem topfartigen Düseneinsatz 20 den Strömungsweg des Medikaments in dem Zerstäubungsraum 32 definiert. Am Boden des Düseneinsatzes sind innenseitig Nuten ausgebildet, über die das Medikament zu der Düse 21 strömt, um dort zerstäubt bzw. zerwellt zu werden.

In der in Fig. 2 dargestellten Speicherstellung liegen die Dichtlippen 54 und 56 so an der Außenseite des einen Zapfen bildenden Einsatzes 16 an, dass der Querkanal 44 zwischen ihnen angeordnet ist. Zum Applizieren des in dem Aufnahmeraum 48 vorgehaltenen Medikaments 24 wird das Speicherelement 14 durch Ausüben eines stirnseitigen Drucks auf dem Einsatz 16 in Richtung des Sockels 26 des Trägerkörpers 12 verschoben, so dass die Dichtlippe 56 den Querkanal 44 überstreicht und eine Fluidverbindung zwischen dem Aufnahmeraum 48 und dem Querkanal 44 über einen zwischen der Innenwand 52 und dem Umfang des Einsatzes 16 gebildeten Ringraum freigegeben ist. Durch Weiterdrücken bzw. weiteres Verfahren des Speicherelements 14 auf dem Einsatz 16 wird dann das Medikament 24 mittels des Einsatzes 16 aus dem Aufnahmeraum 48 verdrängt, über den Querkanal 44 und den von dem Schaft 62 des Reduzierstabs 60 und dem Einsatz 16 gebildeten Ringraum in den Axialkanal 30 und den von dem Schaft 62 des Reduzierstabs 60 und dem Sockel 26 des Trägerkörpers 12 im Bereich des Austragkanals 30 gebildeten Ringraum zu dem Zerstäubungsraum 32 gefördert und von dort durch die Ausbildung des Kopfes 64 über die Düse 21 in zerstäubter Form ausgetragen. Durch den Reduzierstab 60 kann das Medikament 24 im Wesentlichen rückstandsfrei aus der Austragvorrichtung 10 ausgetragen werden.

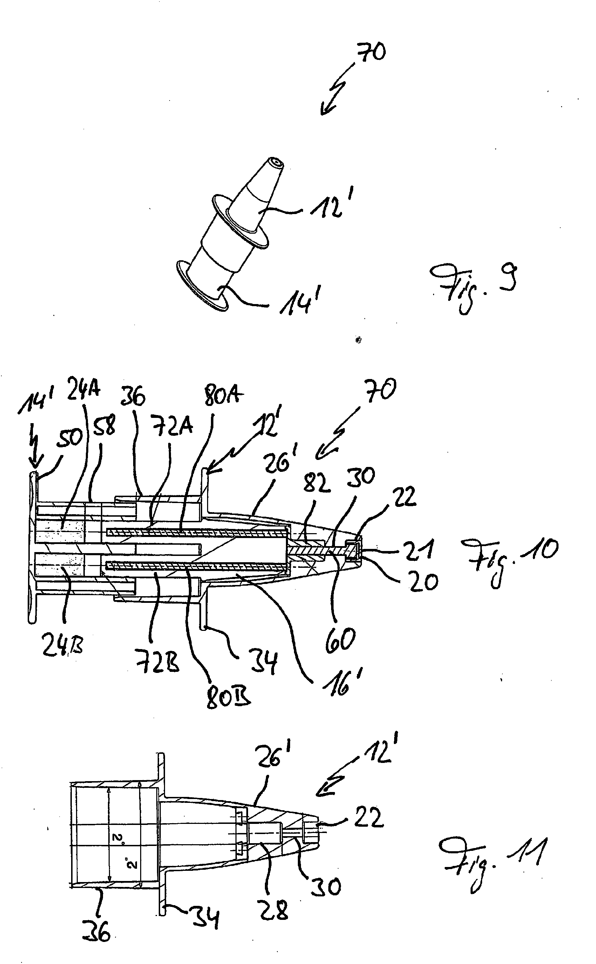

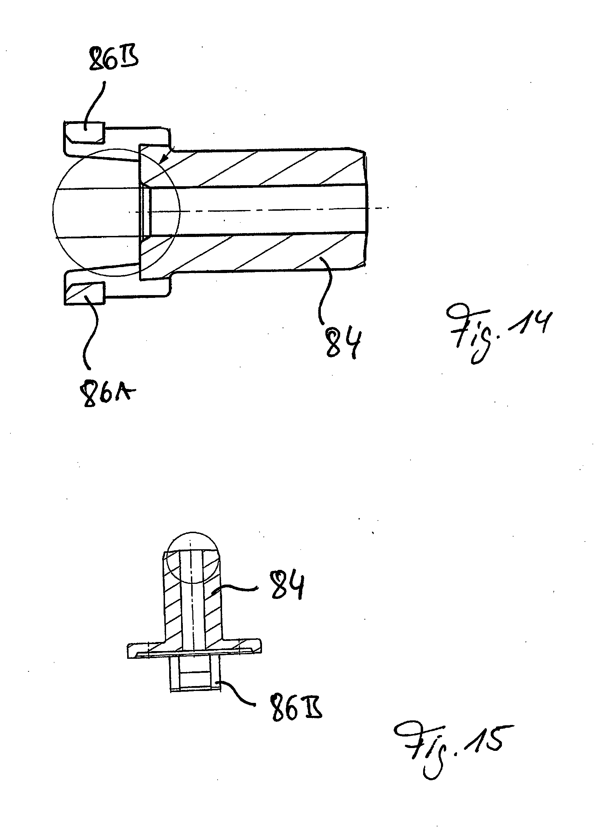

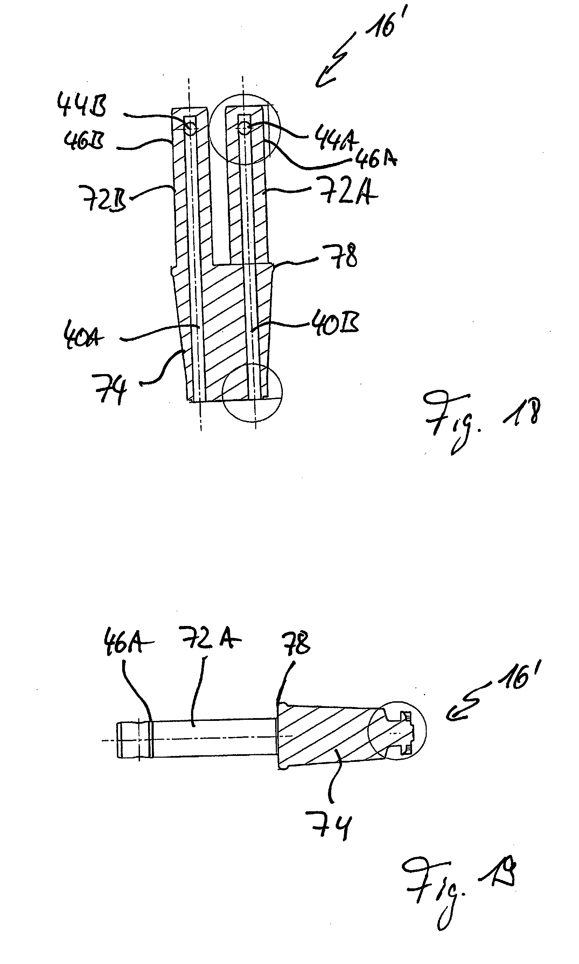

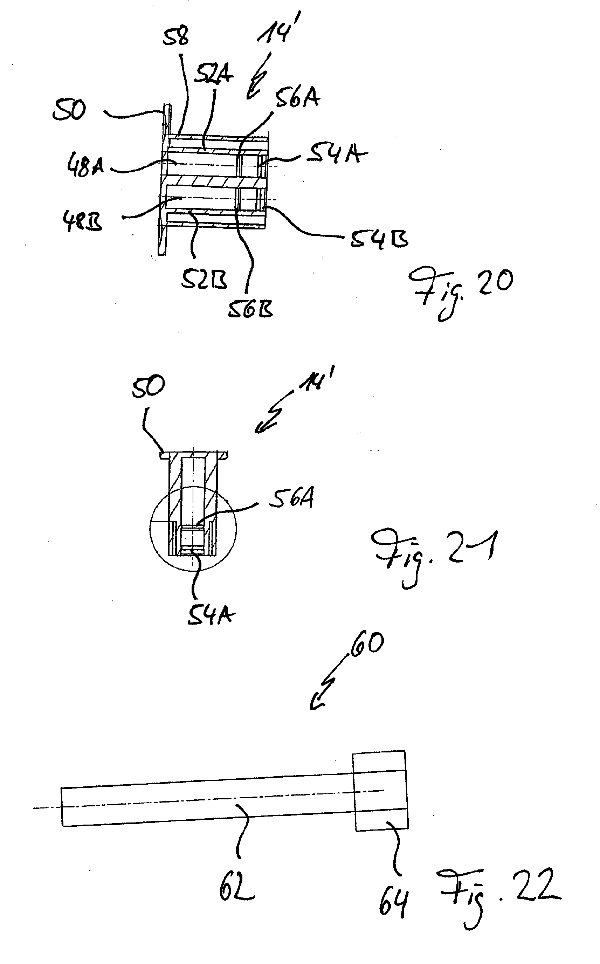

In den Figuren 9 bis 22 ist eine Austragvorrichtung 70 dargestellt, deren äußere Erscheinungsform derjenigen der Austragvorrichtung nach den Figuren 1 bis 8 entspricht. Jedoch ist die Austragvorrichtung 70 zur Applikation eines Medikaments aus zwei Komponenten 24A und 24B geeignet. Hierzu weist die Austragvorrichtung 70 ein als Drücker ausgebildetes Speicherelement 14' auf, das zwei Aufnahmeräume 48A und 48B für jeweils eine Medikamentenkomponente 24A bzw. 24B umfasst, die jeweils von einer kreisförmigen Innenwand 52A bzw. 52B begrenzt sind. Entsprechend der Ausführungsform nach den Figuren 1 bis 8 weist das Speicherelement 14' ebenfalls eine Außenwand 58 auf, die einen im Wesentlichen elliptischen Grundriss hat.

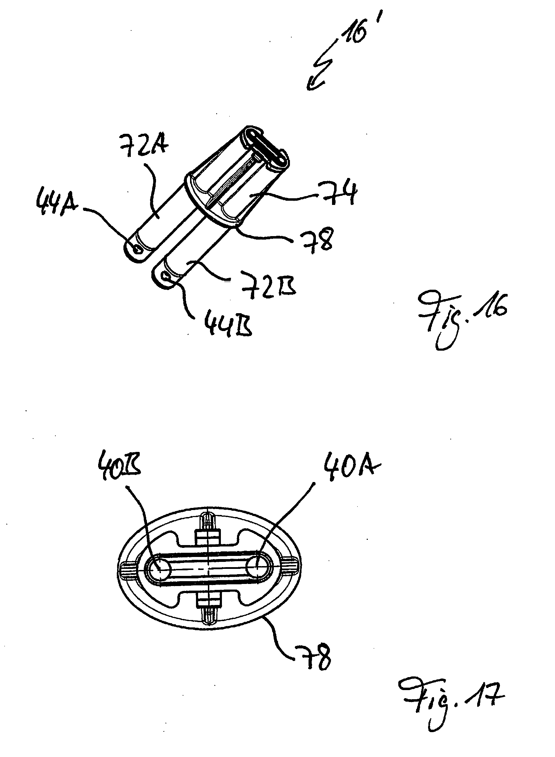

Die beiden Innenwände 52A und 52B des Speicherelements 14' weisen jeweils zwei Dichtlippen 54A und 56A bzw. 54B und 56B auf, die zur Führung des Speicherelements 14' an zwei zylindrischen Zapfen 72A und 72B eines Einsatzes 16' dienen, der in einem Trägerkörper 12' der Austragvorrichtung 70 fixiert ist. Wie den Figuren 16 bis 19 zu entnehmen ist, weist der Einsatz 16' einen als Rastabschnitt dienenden Sockel 74 auf, der in einer Ausnehmung 76 eines Sockels 26' des Trägerkörpers 12' verrastet ist. Hierzu weist der Sockel 74 einen Rastbund 78 auf.

Von dem Sockel 74 zweigen die beiden Zapfen 72A und 72B ab, an denen das Speicherelement 14 geführt ist, und die entsprechend dem in Figur 6 dargestellten Einsatz jeweils eine Lippe 46A bzw. 46B aufweisen, die an der Innenseite der betreffenden Innenwand 52A bzw. 52B des Speicherelements 14' anliegt.

Des Weiteren weisen die beiden Zapfen 72A und 72B des Einsatzes 16' jeweils einen Axialkanal 40A bzw. 40B auf, der auf der dem Speicherelement 14 zugewandten Seite von einer jeweiligen Stirnwand 52A bzw. 52B begrenzt ist und den Sockel 74 bis zu der dem Speicherelement 14' abgewandten Stirnseite durchgreift. Des Weiteren zweigt von den Axialkanälen 40A und 40B jeweils ein Querkanal 44A bzw. 44B ab, dessen Funktion der Funktion des Querkanals des Einsatzes nach Fig. 6 entspricht.

In den Axialkanälen 40A und 40B ist jeweils ein Reduzierstab 80A bzw. 80B angeordnet, der zur Minimierung von Medikamentenrückständen beim Austragen desselben dient.

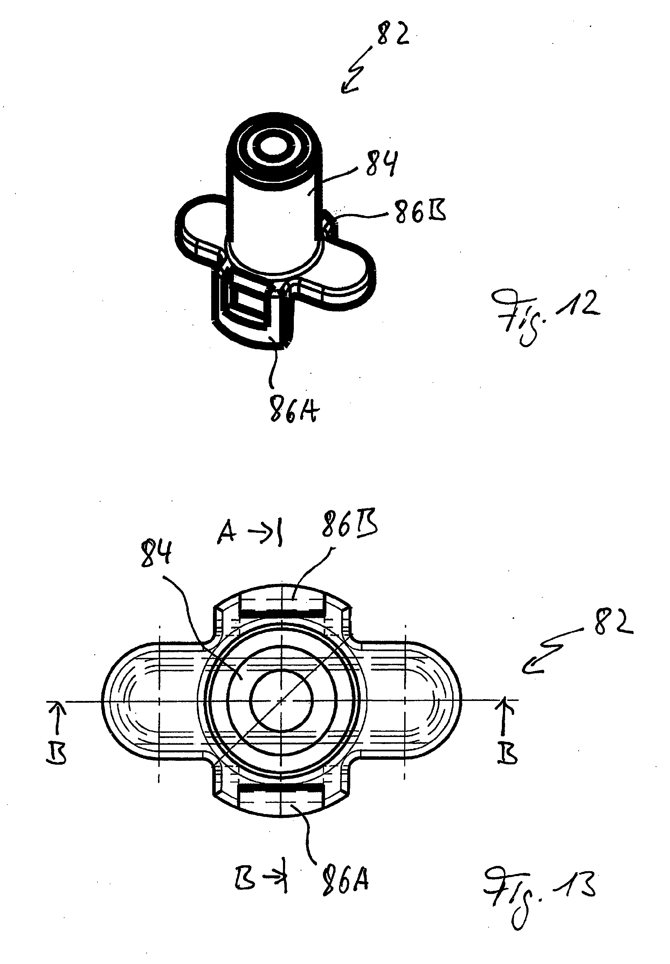

An die dem Speicherelement 14' abgewandte Stirnseite des Einsatzes 16' grenzt ein Leitstück 82, das in den Figuren 12 bis 15 in Alleinstellung dargestellt ist und einen in einen Durchgang 28 des Trägerkörpers 12' eingreifenden Rohrabschnitt 84 und zwei als Rastlaschen ausgebildete Fußabschnitte 86A und 86B aufweist, die mit einem korrespondierenden Kopfabschnitt 88 des Einsatzes 16' verrastbar sind. In dem Leitstück 82 werden die aus den Aufnahmeräumen 48A und 48B ausgetragenen Medikamentenkomponenten 24A und 24B gemischt, um anschließend durch den Rohrabschnitt 84 zu einem stirnseitig in dem Sockel 26 des Trägerkörpers 12' ausgebildeten Zerstäubungsraum 32 gefördert zu werden, der entsprechend dem Zerstäubungsraum der Ausführungsform nach den Figuren 1 bis 8 ausgebildet ist. Ebenfalls entsprechend der Ausführungsform nach den Figuren 1 bis 8 ist an dem Sockel 26' im Bereich einer Austragöffnung 22 ein topfartiger Düseneinsatz 20 eingesetzt, der den Zerstäubungsraum 32 radial begrenzt und eine Düse 21 bildet.

Des Weiteren weist auch die in den Figuren 1 bis 23 dargestellte Ausführungsform einen Reduzierstab 60 auf, der einen hexagonal prismatisch ausgebildeten Kopf 64 und einen Schaft 62 aufweist, wobei der Kopf 64 in dem Zerstäubungsraum 32 und der Schaft 62 in dem Austragkanal 30 und dem Rohrabschnitt 84 des Leitstücks 82 angeordnet ist und zur Innenvolumenreduzierung der Austragvorrichtung 70 dient.

Bei der Applikation des in der Austragvorrichtung 70 vorgehaltenen Medikaments übt ein Nutzer auf die Drückerplatte 50 des Speicherelements 14' Druck aus, so dass die Austragvorrichtung 70 teleskopiert und das Speicherelement 14' auf den Zapfen 72A und 72B des Einsatzes 16' in Richtung des Sockels 26' des Trägerkörpers 12' verfahren wird. Hierbei überstreichen die Dichtlippen 56A und 56B die Querkanäle 44A und 44B der Zapfen 72A und 72B, wodurch durch die Kolbenwirkung der Zapfen 72A und 72B die Komponenten 24A und 24B über die Querkanäle 44A und 44B und die Axialkanäle 40A und 40B zu dem Leitstück 82 gefördert, dort gemischt und dann über den Austragkanal 30 und den Zerstäubungsraum 32 in zerstäubter Form über die Düse 21 ausgetragen werden.

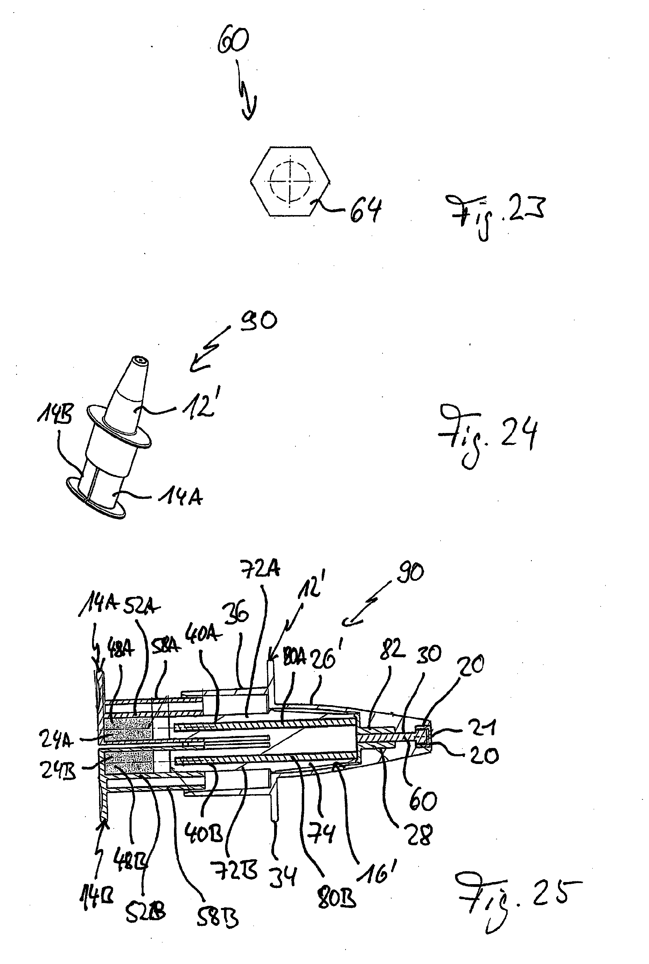

In den Figuren 24 und 25 ist eine weitere Ausführungsform einer Austragvorrichtung 90 dargestellt, deren Aufbau im Wesentlichen demjenigen der Ausführungsform nach den Figuren 9 bis 23 entspricht, sich von dieser aber dadurch unterscheidet, dass sie zwei Speicherelemente 14A und 14B aufweist, die separat auf den beiden Zapfen 72A und 72B verfahren werden können. Die Austragvorrichtung 90 kann beispielsweise dazu genutzt werden, um einen ersten Medikamentteil 24A, der in dem Speicherelement 14A vorgehalten ist, in das eine Nasenloch einer menschlichen Nase und einen zweiten Medikamentteil 24B, der in dem zweiten Speicherelement 14B vorgehalten ist, in das zweite Nasenloch einer menschlichen Nase zu applizieren.

Das Speicherelement 14A, das dem Zapfen 72A zugeordnet ist, weist einen Aufnahmeraum 48A für den Medikamentteil 24A auf, und das Speicherelement 14B, das dem Zapfen 72B zugeordnet ist, weist einen Aufnahmeraum 48B für den zweiten Medikamentteil 24B auf. Die beiden Aufnahmeräume 48A und 48B sind jeweils von einer zylindrischen Innenwand 52A bzw. 52B begrenzt, die an dem jeweils zugeordneten Zapfen 72A bzw. 72B geführt ist und von einer Außenwand 58A bzw. 58B begrenzt ist, die einen halbelliptischen Grundriss hat. Entsprechend sind die Aufnahmeräume 48A und 48B stirnseitig jeweils von einer Drückerplatte 50A bzw. 50B begrenzt, die eine halbelliptische Grundfläche hat.

Im Übrigen entspricht der Aufbau der Austragvorrichtung 90 im Wesentlichen demjenigen der Austragvorrichtung nach den Figuren 9 bis 23.

### Bezugszeichenliste

- 10: Austragvorrichtung
- 12, 12': Trägerkörper
- 14, 14', 14A, 14B: Speicherelement
- 16, 16': Einsatz
- 20: Düseneinsatz
- 21: Düse
- 22: Austragöffnung
- 24, 24A, 24B: Medikament
- 26, 26': Sockel
- 28: Durchgang
- 30: Austragkanal
- 32: Zerstäubungsraum
- 34: Fingeranlageplatte
- 36: Führungswand
- 38: Ringbund
- 40, 40A, 40B: Axialkanal
- 42: Stirnwand
- 44, 44A, 44B: Querkanal
- 46, 46A, 46B: Lippe
- 48, 48A, 48B: Aufnahmeraum
- 50, 50A, 50B: Drückerplatte
- 52, 52A, 52B: Innenwand
- 54, 54A, 54B: Dichtlippe
- 56, 56A, 56B: Dichtlippe
- 58, 58A, 58B: Außenwand
- 60: Reduzierstab
- 62: Schaft
- 64: Kopf
- 70: Austragvorrichtung
- 72A, 72B: Zapfen
- 74: Sockel
- 76: Ausnehmung
- 78: Rastbund
- 80A, 80B: Reduzierstab
- 82: Leitstück
- 84: Rohrabschnitt
- 86A, 86B: Fußabschnitt
- 90: Austragvorrichtung

## Patentansprüche

1. Austragvorrichtung für fließfähigen Stoff, umfassend mindestens ein Speicherelement (14, 14A, 14B) mit mindestens einem Aufnahmeraum (48; 48A, 48B) für fließfähigen Stoff, einen Trägerkörper (12) an dem eine Austragöffnung (22) ausgebildet ist und der mit mindestens einem zumindest bereichsweise zylindrischen Zapfen (16; 72A, 72B) versehen ist, der einen Axialkanal (40; 40A, 40B) und mindestens einen von dem Axialkanal (40; 40A, 40B) abzweigenden Querkanal (44; 44A, 44B) aufweist und an dessen Außenseite das Speicherelement (14) über mindestens eine ringförmige Dichtlippe (54, 56; 54A, 56A, 54B, 56B) geführt ist und an den stirnseitig der Aufnahmeraum (48; 48A, 48B) des Speicherelements (14; 14A, 14B) grenzt, wobei ein Austragen des fließfähigen Stoffes durch ein Verschieben des Speicherelements (14; 14A, 14B) auf den zylindrischen Zapfen (16; 72A, 72B) in Richtung der Austragöffnung (22) erfolgt, so dass die Dichtlippe (54, 56; 54A, 56A, 54B, 56B) den Querkanal (44; 44A, 44B) des zylindrischen Zapfens (16; 72A, 72B) überstreicht und der zylindrische Zapfen (16; 72A, 72B) fließfähigen Stoff nach Art eines Verdrängerkolbens aus dem Aufnahmeraum (48; 48A, 48B) über den Querkanal (44; 44A, 44B) und den Axialkanal (40; 40A, 40B) zu der Austragöffnung (22) fördert und fließfähiger Stoff ausgetragen wird, wobei der Trägerkörper (12) im Bereich der Austragöffnung (22) eine Zerstäubungseinrichtung umfasst, mittels der der fließfähige Stoff durch Verschieben des Speicherelements (14; 14A, 14B) in zerstäubter Form ausgetragen wird, **dadurch gekennzeichnet, dass** die Zerstäubungseinrichtung eine Düse (21) umfasst, die stromab eines Zerstäubungsraums (32) angeordnet ist, in dem ein Ablenkelement (64) angeordnet ist, so dass fließfähiger Stoff in zerstäubter Form aus der Düse (21) ausgetragen wird, und dass stromauf des Zerstäubungsraums (32) ein Austragkanal (30) angeordnet ist, in den ein Reduzierstab (60) eingreift.

2. Austragvorrichtung nach Anspruch 1, **dadurch gekennzeichnet, dass** der Reduzierstab (60) einstückig mit dem Ablenkelement (64) ausgebildet ist.

3. Austragvorrichtung nach einem der Ansprüche 1 bis 2, **dadurch gekennzeichnet, dass** der zylindrische Zapfen (16; 72A, 72B) von einem Einsatz (16) gebildet ist, der an dem Trägerkörper (12) insbesondere über einen Ringbund (38; 78) verrastet ist.

4. Austragvorrichtung nach einem der Ansprüche 1 bis 3, **dadurch gekennzeichnet, dass** der Axialkanal (40) des Zapfens (16) mit dem Austragkanal (30) fluchtet.

5. Austragvorrichtung nach einem der Ansprüche 1 bis 4, **dadurch gekennzeichnet, dass** die Düse (21) an einem topfartigen Düseneinsatz (20) ausgebildet ist, der den Zerstäubungsraum (32) radial begrenzt.

6. Austragvorrichtung nach Anspruch 1, **dadurch gekennzeichnet, dass** der Reduzierstab (60) in den Axialkanal (30) des Zapfens (16) eingreift und diesen vorzugsweise weitgehend durchgreift.

7. Austragvorrichtung nach Anspruch 3, **dadurch gekennzeichnet, dass** der Trägerkörper (12) einen axialen Durchgang (28) hat, der gestuft ausgebildet ist und in den der Einsatz (16) eingreift.

8. Austragvorrichtung nach einem der Ansprüche 1 bis 7, **dadurch gekennzeichnet, dass** an dem Einsatz (16) mindestens zwei Zapfen (72A, 82B) ausgebildet sind, an denen jeweils ein Speicherelement (14A, 14B) verschiebbar geführt ist.

9. Austragvorrichtung nach einem der Ansprüche 1 bis 7, **dadurch gekennzeichnet, dass** an dem Einsatz (16') zwei Zapfen (72A, 72B) ausgebildet sind, an denen das Speicherelement (14) mit zwei Aufnahmeräumen (48A, 48B) verschiebbar geführt ist und die jeweils in einen der Aufnahmeräume (48A, 48B) eingreifen.

10. Austragvorrichtung nach Anspruch 8 oder 9, **dadurch gekennzeichnet, dass** in den Axialkanälen (40A, 40B) der Zapfen (72A, 72B) jeweils ein Reduzierstab (80A, 80B) angeordnet ist.

11. Austragvorrichtung nach einem der Ansprüche 1 bis 10, **dadurch gekennzeichnet, dass** stromab des Einsatzes (16') ein Leitstück (82) angeordnet ist, das vorzugsweise zumindest teilweise den zu dem Zerstäubungsraum (32) führenden Austragkanal (30) bildet.

12. Austragvorrichtung nach einem der Ansprüche 1 bis 11, **dadurch gekennzeichnet, dass** an dem Trägerkörper (12) mindestens eine Fingeranlagefläche (34) ausgebildet ist.

13. Austragvorrichtung nach einem der Ansprüche 1 bis 12, **dadurch gekennzeichnet, dass** das Speicherelement (14) eine Drückerplatte (50) aufweist.

## Claims

1. A discharge device for a flowable substance, comprising at least one storage element (14, 14A, 14B) having at least one reception chamber (48; 48A, 48B) for a flowable substance, a carrier body (12), at which a discharge opening (22) is formed and which is provided with at least one pin (16; 72A, 72B), which is cylindrical at least in portions and which has an axial channel (40; 40A, 40B) and at least one transverse channel (44; 44A, 44B) branching off from the axial channel (40; 40A, 40B) and on the outer side of which the storage element (14) is guided via at least one annular sealing lip (54, 56; 54A, 56A, 54B, 56B) and to which the reception chamber (48; 48A, 48B) of the storage element (14; 14A, 14B) adjoins at the front side, wherein a discharge of the flowable substance is effected by sliding the storage element (14; 14A, 14B) onto the cylindrical pin (16; 72A, 72B) in the direction of the discharge opening (22) so that the sealing lip (54, 56; 54A, 56A, 54B, 56B) skims over the transverse channel (44; 44A, 44B) of the cylindrical pin (16; 72A, 72B) and the cylindrical pin (16; 72A, 72B) transports the flowable substance in the manner of a displacement piston out of the reception chamber (48; 48A, 48B) via the transverse channel (44; 44A, 44B) and the axial channel (40; 40A, 40B) towards the discharge opening (22) and the flowable substance is discharged, wherein the carrier body (12), in the area of the discharge opening (22), comprises an atomizing device, by means of which the flowable substance is discharged in atomized form by a sliding of the storage element (14; 14A, 14B),
**characterized in that**
the atomizing device comprises a nozzle (21), which is arranged downstream of an atomizing chamber (32), in which a deflecting element (64) is arranged so that the flowable substance is discharged from the nozzle (21) in atomized form, and that upstream of the atomizing chamber (32), a discharge channel (30) is arranged, which is engaged by a reduction rod (60).

2. The discharge device according to claim 1,
**characterized in that**
the reduction rod (60) is formed integrally with the deflecting element (64).

3. The discharge device according to any of the claims 1 to 2,
**characterized in that**
the cylindrical pin (16; 72A, 72B) is formed by an insert (16), which is locked to the carrier body (12) in particular via an annular shoulder (38; 78).

4. The discharge device according to any of the claims 1 to 3,
**characterized in that**
the axial channel (40) of the pin (16) is aligned with the discharge channel (30).

5. The discharge device according to any of the claims 1 to 4,
**characterized in that**
the nozzle (21) is formed at a pot-shaped nozzle insert (20), which radially delimits the atomizing chamber (32).

6. The discharge device according to claim 1,
**characterized in that**
the reduction rod (60) engages the axial channel (30) of the pin (16) and preferably penetrates the latter to a large extent.

7. The discharge device according to claim 3,
**characterized in that**
the carrier body (12) has an axial passage (28), which is formed in a stepped manner and into which the insert (16) engages.

8. The discharge device according to any of the claims 1 to 7,
**characterized in that**
on the insert (16), at least two pins (72A, 72B) are formed, at each of which one storage element (14A, 14B) is slidably guided.

9. The discharge device according to any of the claims 1 to 7,
**characterized in that**
on the insert (16'), two pins (72A, 72B) are formed, at which the storage element (14) having two reception chambers (48A, 48B) is slidably guided and each of which engages one of the reception chambers (48A, 48B).

10. The discharge device according to claim 8 or 9,
**characterized in that**
in each of the axial channels (40A, 40B) of the pins (72A, 72B), one reduction rod (80A, 80B) is arranged.

11. The discharge device according to any of the claims 1 to 10,
**characterized in that**
downstream of the insert (16'), a guiding piece (82) is arranged, which preferably forms at least in part the discharge channel (30) leading to the atomizing chamber (32).

12. The discharge device according to any of the claims 1 to 11,
**characterized in that**
on the carrier body (12), at least one finger rest surface (34) is formed.

13. The discharge device according to any of the claims 1 to 12,
**characterized in that**
the storage element (14) comprises a plunger plate (50).

## Revendications

1. Dispositif d'évacuation pour une matière fluide, comprenant au moins un élément de stockage (14, 14A, 14B) ayant au moins une chambre de logement (48 ; 48A, 48B) pour la matière fluide, un corps de support (12) sur lequel est formé un orifice d'évacuation (22) et qui est muni d'un bouchon (16 ; 72A, 72B) au moins par endroits cylindrique qui a un canal axial (40 ; 40A, 40B) et au moins un canal transversal (44 ; 44A, 44B) bifurquant du canal axial (40 ; 40A, 40B) et sur la face extérieure duquel est guidé l'élément de stockage (14) par au moins une lèvre d'étanchéité (54, 56 ; 54A, 56A, 54B, 56B) annulaire et auquel jouxte la chambre de logement (48 ; 48A, 48B) de l'élément de stockage (14 ; 14A, 14B) du côté frontal, dans lequel une évacuation de la matière fluide s'effectue par un déplacement de l'élément de stockage (14 ; 14A, 14B) sur le bouchon cylindrique (16 ; 72A, 72B) en direction de l'orifice d'évacuation (22) de telle sorte que la lèvre d'étanchéité (54, 56 ; 54A, 56A, 54B, 56B) effleure le canal transversal (44 ; 44A, 44B) du bouchon cylindrique (16 ; 72A, 72B) et le bouchon cylindrique (16 ; 72A, 72B) transporte la matière fluide à la manière d'un piston de déplacement à partir de la chambre de logement (48 ; 48A, 48B) par le canal transversal (44 ; 44A, 44B) et par le canal axial (40 ; 40A, 40B) jusqu'à l'orifice d'évacuation (22) et la matière fluide est évacuée, dans lequel le corps de support (12) comprend un dispositif de pulvérisation dans la zone de l'orifice d'évacuation (22), par lequel la matière fluide est évacuée sous forme pulvérisée par le déplacement de l'élément de stockage (14 ; 14A, 14B),
**caractérisé en ce que**
le dispositif de pulvérisation comprend une buse (21) qui est disposée en aval d'une chambre de pulvérisation (32) dans laquelle un élément de déflexion (64) est disposé de telle sorte que la matière fluide est évacuée de la buse (21) sous forme pulvérisée, et qu'en amont de la chambre de pulvérisation (32) un canal d'évacuation (30) est agencé qui est engagé par une tige de réduction (60).

2. Dispositif d'évacuation selon la revendication 1,
**caractérisé en ce que**
la tige de réduction (60) est formée d'un seul tenant avec l'élément de déflexion (64).

3. Dispositif d'évacuation selon l'une quelconque des revendications 1 à 2,
**caractérisé en ce que**
le bouchon cylindrique (16 ; 72A, 72B) est formé par un insert (16) qui est enclenché au corps de support (12) particulièrement par une collerette annulaire (38 ; 78).

4. Dispositif d'évacuation selon l'une quelconque des revendications 1 à 3,
**caractérisé en ce que**
le canal axial (40) du bouchon (16) est aligné avec le canal d'évacuation (30).

5. Dispositif d'évacuation selon l'une quelconque des revendications 1 à 4,
**caractérisé en ce que**
la buse (21) est formée sur un insert de buse (20) en forme de pot qui délimite radialement la chambre de pulvérisation (32).

6. Dispositif d'évacuation selon la revendication 1,
**caractérisé en ce que**
la tige de réduction (60) engage dans le canal axial (30) du bouchon (16) et le pénètre, de préférence, en grande partie.

7. Dispositif d'évacuation selon la revendication 3,
**caractérisé en ce que**
le corps de support (12) a un passage axial (28) qui est formé étagé et dans lequel engage l'insert (16).

8. Dispositif d'évacuation selon l'une quelconque des revendications 1 à 7,
**caractérisé en ce que**
sur l'insert (16), au moins deux bouchons (72A, 82B) sont formés sur lesquels, respectivement, un élément de stockage (14A, 14B) est guidé de manière déplaçable.

9. Dispositif d'évacuation selon l'une quelconque des revendications 1 à 7,
**caractérisé en ce que**
sur l'insert (16'), deux bouchons (72A, 72B) sont formés sur lesquels l'élément de stockage (14) est guidé avec deux chambres de logement (48A, 48B) de manière déplaçable et qui engagent respectivement dans une des chambres de logement (48A, 48B).

10. Dispositif d'évacuation selon les revendications 8 ou 9,
**caractérisé en ce qu'**
une tige de réduction (80A, 80B) est disposée respectivement dans les canaux axiaux (40A, 40B) des bouchons (72A, 72B).

11. Dispositif d'évacuation selon l'une quelconque des revendications 1 à 10,
**caractérisé en ce qu'**
en aval de l'insert (16'), une pièce de guidage (82) est disposée qui forme, de préférence, au moins en partie le canal d'évacuation (30) conduisant à la chambre de pulvérisation (32).

12. Dispositif d'évacuation selon l'une quelconque des revendications 1 à 11,
**caractérisé en ce qu'**
au moins une surface d'appui digital (34) est formée sur le corps de support (12).

13. Dispositif d'évacuation selon l'une quelconque des revendications 1 à 12,
**caractérisé en ce que**
l'élément de stockage (14) comprend une plaque à pression (50).
